# EUROPEAN PATENT SPECIFICATION

(11) **EP 4 014 938 B1**
(45) Date of publication and mention of the grant of the patent: **05.03.2025**
(21) Application number: 20872101.9
(22) Date of filing: 11.06.2020
(51) Int. Cl.: A61F 13/532, A61F 13/533

(54) **ABSORBENT ARTICLE**
ABSORBIERENDER ARTIKEL
ARTICLE ABSORBANT

(30) Priority: 30.09.2019 JP 2019180591
(43) Date of publication of application: 22.06.2022
(73) Proprietor: Unicharm Corporation, Shikokuchuo-shi, Ehime 799-0111 (JP)
(72) Inventor: ONISHI, Kazuaki, Kanonji-shi, Kagawa 769-1602 (JP); TSUKUDA, Atsushi, Kanonji-shi, Kagawa 769-1602 (JP); TADA, Hiroaki, Kanonji-shi, Kagawa 769-1602 (JP)
(74) Representative: Dolleymores
(86) International application number: PCT/JP2020/023101
(87) International publication number: WO 2021/065084

(56) References cited:
- EP-A1- 3 482 731
- WO-A1-2013/187375
- WO-A1-2013/187376
- WO-A1-2019/158238
- JP-A- 2014 014 667
- JP-A- 2017 070 497
- JP-A- 2018 000 872
- JP-A- 2019 118 722

## Description

### FIELD

The present invention relates to an absorbent article.

### BACKGROUND

There are known absorbent articles that exhibit excellent absorption performance (for example, absorption rate, and liquid return suppression) even when the absorbent articles repeatedly absorb bodily fluids. For example, Patent Literature 1 discloses an absorbent article including an absorbent core including superabsorbent polymer particles. In the absorbent article, the absorbent core is formed of a first layer on a non-skin facing surface side and a second layer on a skin facing surface side. The first layer includes a plurality of groove portions including a plurality of main groove portions that extend in a longitudinal direction and penetrate the first layer in a thickness direction, and a plurality of base portions that extend in the longitudinal direction, and each of the plurality of main groove portions and each of the plurality of base portions are arranged alternately in a lateral direction. The second layer includes a plurality of main groove portion corresponding portions and a plurality of base corresponding portions arranged at positions overlapping with the plurality of main groove portions and the plurality of base portions in the thickness direction, respectively. Both an average density of the superabsorbent polymer particles contained in each of the plurality of base portions and an average density of the superabsorbent polymer particles contained in each of the plurality of main groove portion corresponding portions are lower than an average density of the superabsorbent polymer particles contained in each of the plurality of base corresponding portions.

### [CITATION LIST]

### [PATENT LITERATURE]

Patent Literature 1: Japanese Unexamined Patent Publication No. 2018-872 WO 2013/187375 A1 discloses a further prior art example of an absorbent article including an absorbent core including superabsorbent polymer particles.

### SUMMARY

### [TECHNICAL PROBLEM]

An absorbent article as described in Patent Literature 1 includes a main groove portion and a base portion adjacent to the main groove portion in the first layer on the non-skin facing surface side. Therefore, the bodily fluid (for example: urine) that has reached the first layer through the second layer can be diffused in the main groove portion in the longitudinal direction, can penetrate into the base portion and diffuse in the lateral direction, and thus can be held in the base portion. Due to that, the entire absorbent core can be effectively used for absorption of the bodily fluid. Further, since the superabsorbent polymer particles are included in the base portion and swell by absorbing the bodily fluid, the shape of the main groove portion in the thickness direction can be maintained. Therefore, such an absorbent article has excellent absorption performance, for example, diffusibility of bodily fluid, absorption rate, and liquid return suppression, even when the absorbent article repeatedly absorbs the bodily fluid.

However, in such an absorbent article, as the number of times the absorbent article absorbs a bodily fluid increases, the degree of swelling of the superabsorbent polymer particles in the base portion becomes excessively large, and there is a risk that the swollen superabsorbent polymer particles may block the bodily fluid (blocking). In such a case, since the bodily fluid is less likely to penetrate into the base portion and diffuse into the base portion in the lateral direction, there is a risk that the bodily fluid may not be sufficiently absorbed by the absorbent core to cause the deterioration of the absorption performance of the absorbent article, for example, absorption rate, and liquid return suppression. Therefore, there is room for improvement in absorption performance in a case where a bodily fluid is repeatedly absorbed in an absorbent article, for example, absorption rate, and liquid return suppression.

An object of the present invention is to provide an absorbent article capable of improving absorption performance, for example, absorption rate, and liquid return suppression in a case where a bodily fluid is repeatedly absorbed in the absorbent article.

### [SOLUTION TO PROBLEM]

According to the present invention, there is provided an absorbent article as claimed in claim 1. The dependent claims set out preferred but optional features.

### [ADVANTAGEOUS EFFECT OF THE INVENTION]

According to the present invention, an absorbent article capable of improving absorption performance, for example, absorption rate, and liquid return suppression in a case where a bodily fluid is repeatedly absorbed in the absorbent article can be provided.

### BRIEF DESCRIPTION OF DRAWINGS

FIG. 1 is a plan view showing a configuration example of an absorbent article according to an embodiment not in accordance with the invention.
FIG. 2 is a plan view showing a configuration example of an absorbent body of the absorbent article according to the embodiment.
FIG. 3 is a plan view showing a configuration example of an absorbent core of the absorbent article according to the embodiment.
FIG. 4 is a cross-sectional view taken along a line IV-IV in FIG. 2 according to the embodiment.
FIG. 5 is a cross-sectional view taken along a line V-V in FIG. 2 according to the embodiment.
FIG. 6 is a schematic view showing flow of a bodily fluid in the cross section taken along the line IV-IV in FIG. 2 according to the embodiment.
FIG. 7 is a cross-sectional view taken along the line IV-IV in FIG. 2 according to another embodiment.
FIG. 8 is a cross-sectional view taken along the line V-V in FIG. 2 according to another embodiment.
FIG. 9 is a schematic view showing flow of a bodily fluid in the cross section taken along the line IV-IV in FIG. 2 according to another embodiment.
FIG. 10 is a schematic view showing a configuration example of a manufacturing apparatus used in a method of manufacturing the absorbent article according to the embodiment.
FIG. 11 is a schematic view showing a state in which an absorbent material is supplied on a suction drum of a manufacturing apparatus.

### DESCRIPTION OF EMBODIMENTS

The present embodiment relates to the following aspects.

### [Aspect 1]

An absorbent article having a longitudinal direction, a lateral direction, and a thickness direction, the absorbent article comprising: an absorbent core including superabsorbent polymer particles, wherein the absorbent core has a non-skin facing surface, and a skin facing surface, and is formed of a first layer on a non-skin facing surface side, and a second layer on a skin facing surface side, the first layer includes: a plurality of groove portions that includes a plurality of main groove portions extending in the longitudinal direction and penetrating the first layer in the thickness direction, and a plurality of base portions that extend in the longitudinal direction, each of the plurality of main groove portions and each of the plurality of base portions are arranged alternately in the lateral direction, each of the plurality of base portions is formed of: a base inner side portion adjacent to the non-skin facing surface side of the second layer, and a base outer side portion adjacent to the non-skin facing surface side of the base inner side portion, and an average density of the superabsorbent polymer particles contained in the base outer side portion is lower than an average density of the superabsorbent polymer particles contained in the base inner side portion.

Since the absorbent article includes the predetermined main groove portions and the predetermined base portions in the first layer, the bodily fluid that has permeated through the second layer is passed through the main groove portions and diffuses in the longitudinal direction while exuding into the base portions in the lateral direction. Here, in the present absorbent article, the average density of the superabsorbent polymer particles contained in the base outer side portion in the base portion of the first layer is lower than the average density of the superabsorbent polymer particles contained in the base inner side portion. Therefore, even in a case where the number of times the absorbent article absorbs the bodily fluid increases and the blocking due to the swelling of the superabsorbent polymer particles in the base inner side portion is likely to occur, the occurrence of blocking due to the swelling of the superabsorbent polymer particles in the base outer side portion can be suppressed. Due to that, the difficulty of the bodily fluid penetrating into the base portion and the difficulty of the bodily fluid diffusing in the base portion in the lateral direction can be suppressed. On the other hand, the collapse of the main groove portion in the thickness direction can be suppressed due to the swelling of the superabsorbent polymer particles in the base inner side portion. Therefore, as for the entire absorbent article, while the bodily fluid can be stably diffused in the main groove portion in the longitudinal direction, the bodily fluid can be stably diffused in the lateral direction. Accordingly, the entire absorbent core can be more effectively used for absorption of the bodily fluid, and the absorption performance, for example, absorption rate, and liquid return suppression in a case where the bodily fluid is repeatedly absorbed in the absorbent article can be enhanced.

The second layer includes: a plurality of main groove portion corresponding portions and a plurality of base corresponding portions arranged at positions overlapping with the plurality of main groove portions and the plurality of base portions in the thickness direction, respectively, and both an average density of the superabsorbent polymer particles contained in each of the plurality of base portions and an average density of the superabsorbent polymer particles contained in each of the plurality of main groove portion corresponding portions are lower than an average density of the superabsorbent polymer particles contained in each of the plurality of base corresponding portions.

In the absorbent article, both the average density of the superabsorbent polymer particles contained in the base portion and the average density of the superabsorbent polymer particles contained in the main groove portion corresponding portion are lower than the average density of the superabsorbent polymer particles contained in the base corresponding portion. Therefore, when bodily fluid is absorbed, the bodily fluid can easily permeate into the main groove portion through the main groove portion corresponding portion, the water flow function of the main groove portion can be secured, and the bodily fluid that cannot be held in the base portion can be easily absorbed in the base corresponding portion. As described above, in the absorbent article, the bodily fluid can be absorbed by a bodily fluid absorption cycle in which the bodily fluid permeates from the main groove portion corresponding portion to the main groove portion (also referred to as "bodily fluid permeation action"), the permeated bodily fluid is drawn from the main groove portion to the adjacent base portion (also referred to as "bodily fluid drawing action"), and when the drawn bodily fluid cannot be held in the base portion, the bodily fluid is sucked up by the base corresponding portion (also referred to as "bodily fluid sucking action"). At this time, in the absorbent article, even when blocking in the base inner side portion is likely to occur, the occurrence of blocking due to the swelling of the superabsorbent polymer particles in the base outer side portion can be suppressed, and thus the bodily fluid absorption cycle can be stably maintained. Accordingly, even in a case where the bodily fluid is repeatedly absorbed in the absorbent article, the absorption performance can be enhanced.

### [Aspect 2]

The absorbent article according to aspect 1, wherein a thickness of the base outer side portion in the thickness direction is in a range of 20% to 80% of a thickness of each of the plurality of base portions in the thickness direction.

In the absorbent article, the thickness of the base outer side portion is in a range of 20% to 80% of the thickness of the base portion. Therefore, in a case where the absorbent article repeatedly absorbs bodily fluid, even when the blocking due to the swelling of the superabsorbent polymer particles in the base inner side portion is likely to occur, the occurrence of blocking due to the swelling of the superabsorbent polymer particles in the base outer side portion can be further suppressed. Therefore, the difficulty of the bodily fluid penetrating into the base portion and the difficulty of the bodily fluid diffusing in the base portion in the lateral direction can be more stably suppressed.

### [Aspect 3]

The absorbent article according to aspect 1 or 2, wherein the average density of the superabsorbent polymer particles contained in each of the plurality of main groove portion corresponding portions is lower than the average density of the superabsorbent polymer particles contained in the base inner side portion of each of the plurality of base portions.

In the absorbent article according to claim 4, the average density of the superabsorbent polymer particles contained in the main groove portion corresponding portion is lower than the average density of the superabsorbent polymer particles contained in the base inner side portion of the base portion. Therefore, the swelling of the main groove portion corresponding portion can be suppressed, and the inhibition of the bodily fluid permeation action can be suppressed. Therefore, the absorption performance in a case where the bodily fluid is repeatedly absorbed in the absorbent article can be enhanced.

### [Aspect 4]

The absorbent article according to aspect 3,
wherein the average density of the superabsorbent polymer particles contained in the base outer side portion of each of the plurality of base portions is lower than the average density of the superabsorbent polymer particles contained in each of the plurality of main groove portion corresponding portions.

In the absorbent article, the average density of the superabsorbent polymer particles contained in the base outer side portion of the base portion is lower than the average density of the superabsorbent polymer particles contained in the main groove portion corresponding portion. Therefore, the swelling of the base outer side portion can be suppressed, and the inhibition of the bodily fluid drawing action can be suppressed. Therefore, the absorption performance in a case where the bodily fluid is repeatedly absorbed in the absorbent article can be enhanced.

### [Aspect 5]

The absorbent article according to any one of aspects 1 to 4, wherein each of the plurality of groove portions further includes sub-groove portions that penetrate the first layer in the thickness direction and are present in communication with each of the plurality of main groove portions through a base end with a predetermined space in a direction crossing each of the plurality of main groove portions.

In the absorbent article, since the groove portion further includes a sub-groove portion crossing the main groove portion, the diffusion of the bodily fluid in the groove portion can be rapidly performed. Therefore, the entire absorbent core can be more effectively used for absorption of the bodily fluid, and thus the absorption performance in a case where the bodily fluid is repeatedly absorbed in the absorbent article can be enhanced.

### [Aspect 6]

The absorbent article according to aspect 5, wherein the second layer further includes: sub-groove portion corresponding portions arranged at positions overlapping with the sub-groove portions in the thickness direction, and an average density of the superabsorbent polymer particles contained in the sub-groove portion corresponding portion is lower than an average density of the superabsorbent polymer particles contained in each of the plurality of base corresponding portions. In the absorbent article, the average density of the superabsorbent polymer particles contained in the sub-groove portion corresponding portion is lower than the average density of the superabsorbent polymer particles contained in each of the base corresponding portions. Therefore, when bodily fluid is absorbed, the bodily fluid can easily permeate into the sub-groove portion through the sub-groove portion corresponding portion, and the water flow function of the sub-groove portion can be secured. In addition, the bodily fluid can be further absorbed by a bodily fluid absorption cycle in which while being diffused in a direction crossing the longitudinal direction along the sub-groove portion, the bodily fluid is drawn into the base portion and held therein, and the bodily fluid that cannot be held in the base portion is sucked up to the base corresponding portion. Therefore, the absorption performance in a case where the bodily fluid is repeatedly absorbed in the absorbent article can be enhanced.

### [Aspect 7]

The absorbent article according to any one of aspects 1 to 6, wherein in each of the plurality of base portions, at one end portion and the other end portion in the lateral direction, and at a central part between the one end portion and the other end portion, an average density of the superabsorbent polymer particles contained in each of the one end portion and the other end portion is lower than an average density of the superabsorbent polymer particles contained in the central part.

In the absorbent article, in the lateral direction, the average density of the superabsorbent polymer particles contained in both end portions of the base portion is lower than the average density of the superabsorbent polymer particles contained in the central part. Therefore, the swelling of both end portions that come into contact with the main groove portion in the base portion can be suppressed, and the inhibition of the bodily fluid drawing action can be suppressed. Therefore, the absorption performance in a case where the bodily fluid is repeatedly absorbed in the absorbent article can be enhanced.

Hereinafter, an absorbent article according to an embodiment will be described with reference to drawings.
In the present specification, unless otherwise specified, "viewing an object (for example, an absorbent article, an absorbent body, or an absorbent core) placed on a horizontal plane in an unfolded state from above or below the object in the thickness direction" will be referred to as a "plan view". In a case where the object is an absorbent article, viewing the absorbent article in the thickness direction from a top-surface sheet side in an unfolded state may be simply referred to as a "plan view". In a case where the object is an absorbent core, viewing the absorbent article in the thickness direction from a non-skin facing surface side of the absorbent core in an unfolded state may be simply referred to as a "plan view".

Various directions and the like used in the present specification are as follows, unless otherwise specified. The "longitudinal direction" refers to "a direction in which the length of a longitudinally long object in a plan view is long", the "lateral direction" refers to "a direction in which the length of the longitudinally long object in a plan view is short", and the "thickness direction" refers to "a direction vertical to the object placed on a horizontal plane in an unfolded state". The longitudinal direction, the lateral direction, and the thickness direction are in a relationship that is orthogonal to each other.

In the present specification, unless otherwise specified, in the thickness direction of the absorbent article, a "proximal side relative to the skin surface of the wearer while the absorbent article is put on" is referred to as a "skin facing surface side", and a "distal side relative to the skin surface of the wearer while the absorbent article is put on" is referred to as a "non-skin facing surface side". In the present specification, the "skin facing surface side surface" and the "non-skin facing surface side surface" of various members (for example, a top-surface sheet, an absorbent body, and a back-surface sheet) that constitute the absorbent article are simply referred to as a "skin facing surface" and a "non-skin facing surface," respectively.

In the present specification, the "main groove portion" means not only a groove portion that extends in a direction parallel to the longitudinal direction, but also a groove portion that extends along the longitudinal direction. An angle formed between the main groove portion and the longitudinal direction is preferably less than 45°, more preferably less than 30°, and even more preferably less than 15°. The main groove portion can extend linearly, or non-linearly, for example, in a curved shape. In the present specification, the width of the main groove portion means the length of the main groove portion in a direction orthogonal to a direction in which the main groove portion extends in an object portion.

In the present specification, the "sub-groove portion" means not only a groove portion that extends in a direction parallel or substantially parallel to the lateral direction, but also a groove portion that extends in a direction other than the longitudinal direction. An angle formed between the sub-groove portion and the main groove portion (or the tangent line of the main groove portion) at a portion (base end) where the sub-groove portion communicates with the main groove portion is preferably 45° or more, more preferably 60° or more, and even more preferably 80° or more. The sub-groove portion can extend linearly, or non-linearly, for example, in a curved shape. In the present specification, the width of the sub-groove portion means the length of the sub-groove portion in a direction orthogonal to a direction in which the sub-groove portion extends in the object portion.

In the present specification, the terms "front waist region," "back waist region," and "crotch region" are used in a case where the absorbent article is a disposable diaper, and the meanings are as follows. In a pull-on disposable diaper, the front waist region means a region interposed between a pair of joining portions at two lateral end portions that join a front body and a back body in the front body, and the back waist region means a region interposed between the pair of joining portions in the back body. The crotch region means a region between the front waist region and the back waist region. The crotch region also corresponds to a region interposed between the pair of leg openings. In a tape-type disposable diaper, the waist region and the crotch region are partitioned in a fixed state in which the tip ends of a pair of tape fasteners are fixed so as to be adjacent to a predetermined fixing region for the tape fastener. Specifically, the waist region is determined based on a pair of overlapping portions in which a waist forming member of the front body and a waist forming member of the back body overlap with each other in the absorbent article in the above fixed state. The front waist region means a region between the pair of overlapping portions in the front body of the absorbent article. Similarly, the back waist region means a region between the pair of overlapping portions in the back body of the absorbent article. The crotch region means a region between the front waist region and the back waist region.

### <Absorbent article>

### (First embodiment not according to the invention)

Hereinafter, an absorbent article according to an embodiment will be described.

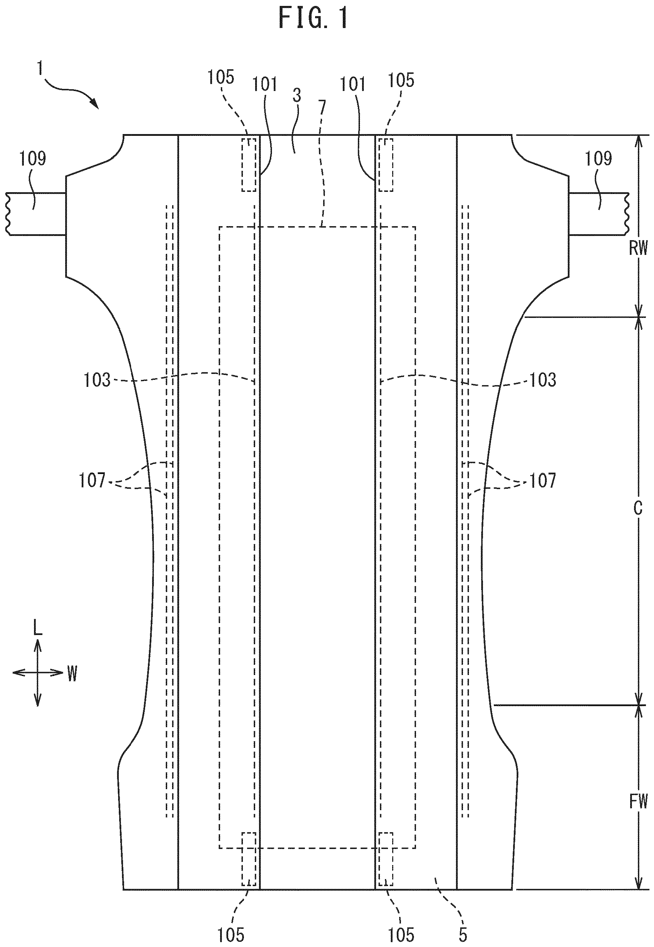

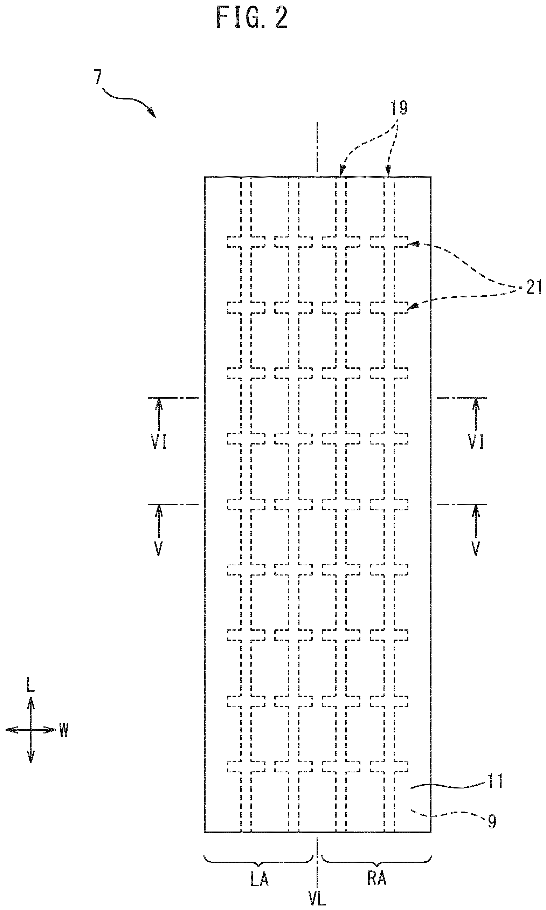

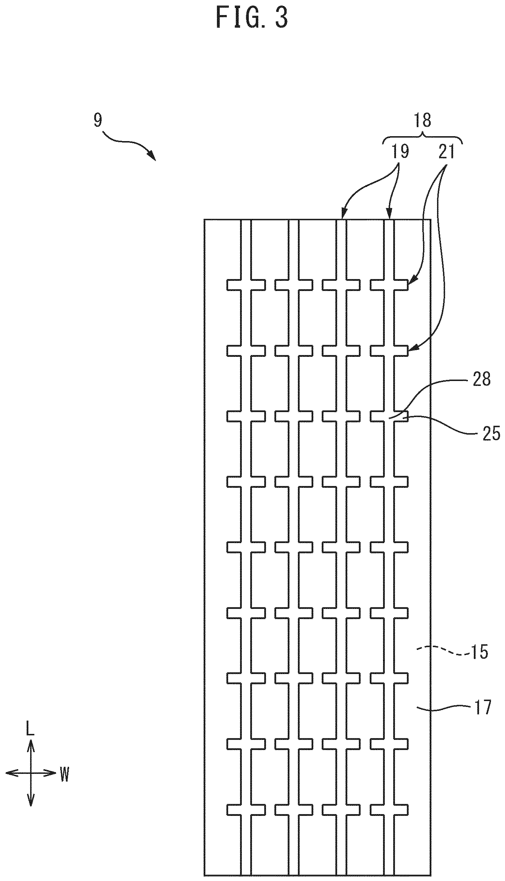

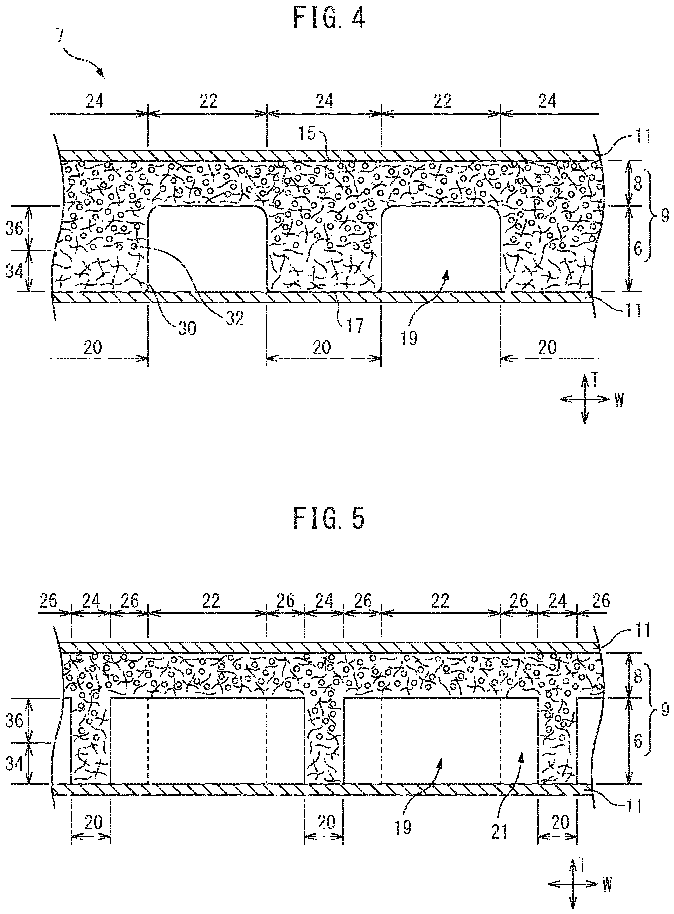

FIGS. 1 to 6 are views showing configuration examples of an absorbent article 1 according to the embodiment, specifically, a disposable diaper. Specifically, FIG. 1 is a plan view showing a configuration example of the absorbent article 1 according to the embodiment. FIG. 2 is a plan view showing a configuration example of an absorbent body 7 of the absorbent article 1 according to the embodiment. FIG. 3 is a plan view showing a configuration example of an absorbent core 9 of the absorbent article 1 according to the embodiment. FIGS. 4 and 5 are a cross-sectional view taken along a line IV-IV in FIG. 2, and a cross-sectional view taken along a line V-V, respectively.

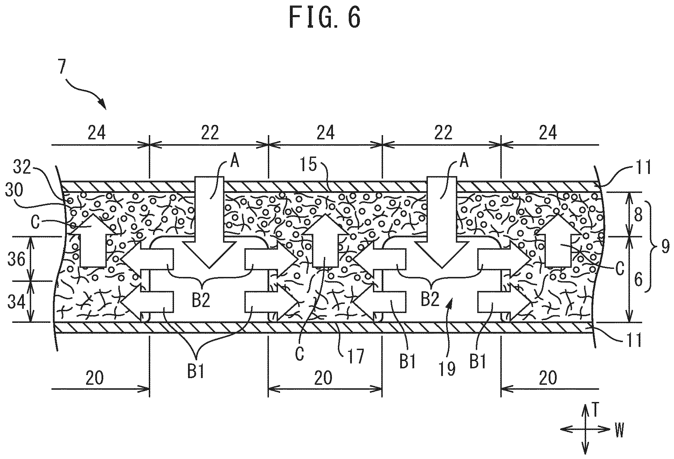

FIG. 6 is a schematic view showing the flow of bodily fluid in the cross section taken along the line IV-IV in FIG. 2.

As shown in FIG. 1, the absorbent article 1 includes a liquid-permeable sheet 3, a liquid-impermeable sheet 5, and an absorbent body 7 arranged between the liquid-permeable sheet 3 and the liquid-impermeable sheet 5. As shown in FIGS. 2 and 3, the absorbent body 7 has a longitudinal direction L, a lateral direction W, and a thickness direction T, and includes an absorbent core 9 having a skin facing surface 15 and a non-skin facing surface 17. In the present embodiment, the absorbent core 9 includes a core wrap 11 formed of a tissue that covers the skin facing surface 15 and the non-skin facing surface 17. It should be noted that, in the present embodiment, since the longitudinal direction, the lateral direction, and the thickness direction of the absorbent article 1 are the same as those of the absorbent body 7, the longitudinal direction L, the lateral direction W, and the thickness direction T are used as the directions of the absorbent article 1.

In the present embodiment, as shown in FIG. 1, the absorbent article 1 further includes a pair of leakproof walls 101 including elastic members 103, fixed portions 105 that fixe the leakproof walls 101 to the liquid-permeable sheet 3, an elastic members 107 around the leg portions, a tape fasteners 109, and the like. It should be noted that these are well-known in the art and will not be described here.

As shown in FIGS. 3 and 4, the absorbent core 9 has the non-skin facing surface 17 and the skin facing surface 15, and is formed of a first layer 6 on the non-skin facing surface side and a second layer 8 on the skin facing surface side. In addition, the first layer 6 includes a plurality of groove portions 18 including a plurality of main groove portions 19 that linearly extend in the longitudinal direction L and penetrate the first layer in the thickness direction T, and a plurality of base portions 20 that extend in the longitudinal direction L, and each of the plurality of main groove portions 19 and each of the plurality of base portions 20 are arranged alternately in the lateral direction W. The main groove portion 19 is recessed from the non-skin facing surface 17 toward the skin facing surface 15 in the thickness direction of the absorbent core 9 and extends in the longitudinal direction L. The base portion 20 is formed of a base inner side portion 36 adjacent to the non-skin facing surface 17 side of the second layer 8, and a base outer side portion 34 adjacent to the non-skin facing surface 17 side of the base inner side portion 36.

In the present embodiment, the second layer 8 includes a plurality of main groove portion corresponding portions 22 at positions that overlap with the plurality of main groove portions 19 in the thickness direction T, and a plurality of base corresponding portions 24 at positions that overlap with the plurality of base portions 20 in the thickness direction T. Further, in the present embodiment, as shown in FIGS. 3 and 5, the groove portion 18 includes a plurality of sub-groove portions 21. In addition, the second layer 8 includes a plurality of sub-groove portion corresponding portions 26 at positions that overlap with the plurality of sub-groove portions 21 in the thickness direction T.

In the present embodiment, the thickness of each of the first layer 6 and the second layer 8 is preferably 0.1 to 5 mm, more preferably 0.5 to 4 mm, and even more preferably 1 to 3 mm. It should be noted that, in the present embodiment, the thickness of each of the first layer 6 and the second layer 8 can be measured in the following non-contact method using the laser displacement meter (for example, high-precision two-dimensional laser displacement meter LJ-G series (model: LJ-G030) manufactured by Keyence Corporation). The absorbent core 9 which is obtained by blowing a cold spray onto the absorbent article 1 so as to peel off the liquid-permeable sheet 3, the liquid-impermeable sheet 5, and the core wrap 11, is cut into a size of 100 mm × 100 mm, and is used as a sample. The sample is placed on a horizontal measurement table in a manner such that the non-skin facing surface 17 having the plurality of groove portions 18 and the plurality of base portions 20 formed thereon faces upward, the displacement from the measurement table is measured by the laser displacement meter for five different base portions 20, and the average value of the five measurement values is set as Ax (mm). Similarly, the displacement from the measurement table is measured by the laser displacement meter for five different groove portions 18 (main groove portions 19), and the average value of the five measurement values is set as Ay (mm). The thickness of the second layer 8 is Ay (mm), and the thickness of the first layer 6 is calculated from a difference between Ax (mm) and Ay (mm).

The absorbent core 9 includes water-absorbent fibers 30 and superabsorbent polymer particles (SAP) 32, and has a function of absorbing and holding the bodily fluid discharged to the absorbent article 1. In the present embodiment, the average basis weight of the absorbent fibers in both the first layer 6 and the second layer 8 is preferably 50 to 250 g/m², and more preferably 80 to 200 g/m². In addition, the average basis weight of the superabsorbent polymer particles in the first layer 6 is smaller than the average basis weight of the superabsorbent polymer particles in the second layer 8. The average basis weight of the superabsorbent polymer particles in the first layer 6 is preferably 20 to 150 g/m², and more preferably 30 to 100 g/m², and the average basis weight of the superabsorbent polymer particles in the second layer 8 is preferably 50 to 350 g/m², and more preferably 100 to 250 g/m². A ratio of the average basis weight of the superabsorbent polymer particles in the first layer 6 to the average basis weight of the superabsorbent polymer particles in the entire absorbent core 9 is preferably 20% to 45% and more preferably 25% to 35%.

As shown in FIG. 4, in the absorbent core 9, the base outer side portion 34 of the base portion 20 and the base inner side portion 36 of the base portion 20 each have different average densities of the superabsorbent polymer particles. Specifically, the average density of the superabsorbent polymer particles contained in the base outer side portion 34 is lower than the average density of the superabsorbent polymer particles contained in the base inner side portion 36. Further, in the present embodiment, the average density of the superabsorbent polymer particles contained in the base inner side portion 36 is equal to the average density of the superabsorbent polymer particles contained in the second layer 8 or lower than the average density of the superabsorbent polymer particles contained in the second layer 8.

As described above, in the present embodiment, the average densities of the superabsorbent polymer particles have a relationship of the base outer side portion 34 < the base inner side portion 36 ≤ the second layer 8. In such a configuration, for example, as shown in FIG. 6, in the present embodiment, the bodily fluid moves in the absorbent core 9 in substantially the following order.
(i) The bodily fluid that has reached the core wrap 11 passes through the second layer and then mainly moves to the main groove portion 19 by gravity (arrow A).
(ii) The bodily fluid that has moved to the main groove portion 19 diffuses in the longitudinal direction L while exuding into the base outer side portion 34 and the base inner side portion 36 of the base portion 20 that are adjacent in the lateral direction W (arrows B1 and B2).
(iii) The bodily fluid that cannot be held by the base portion 20 can be sucked up and held in the base corresponding portion 24 (arrow C).

Since the absorbent article 1 includes the predetermined main groove portion 19 and the predetermined base portion 20 in the first layer 6, the bodily fluid that has permeated through the second layer 8 is passed through the main groove portion 19 and diffuses in the longitudinal direction L while exuding into the base portion 20 in the lateral direction W. Here, in the absorbent article 1, the average density of the superabsorbent polymer particles contained in the base outer side portion 34 of the base portion 20 of the first layer 6 is lower than the average density of the superabsorbent polymer particles contained in the base inner side portion 36. Therefore, even when the number of times the absorbent article 1 absorbs the bodily fluid increases and the blocking due to the swelling of the superabsorbent polymer particles in the base inner side portion 36 is likely to occur (the arrow B2 stops), the occurrence of blocking due to the swelling of the superabsorbent polymer particles in the base outer side portion 34 can be suppressed. That is, regardless of the increase in the number of times of absorption of the bodily fluid, a state in which the bodily fluid moved to the main groove portion 19 easily continuously exudes into the base outer side portion 34 can be maintained (arrow B1 continues). Due to that, the difficulty of the bodily fluid penetrating into the base portion 20 and the difficulty of the bodily fluid diffusing in the base portion 20 in the lateral direction W can be suppressed. On the other hand, the collapse of the main groove portion 19 in the thickness direction T can be suppressed due to swelling of the superabsorbent polymer particles in the base inner side portion 36. Therefore, as for the entire absorbent article 1, while the bodily fluid can be stably diffused in the main groove portion 19 in the longitudinal direction L, the bodily fluid can be stably diffused in the lateral direction W. Accordingly, the entire absorbent core 9 can be more effectively used for absorption of the bodily fluid, and the absorption performance, for example, absorption rate, and liquid return suppression in a case where the bodily fluid is repeatedly absorbed in the absorbent article 1 can be enhanced.

In the present embodiment, the average density of the superabsorbent polymer particles contained in the base outer side portion 34 is preferably 0 to 0.15 g/cm³, more preferably 0 to 0.1 g/cm³, and even more preferably 0 to 0.08 g/cm³. In a case where the average density of the superabsorbent polymer particles contained in the base outer side portion 34 is excessively high, the blocking due to the swelling of the superabsorbent polymer particles in the base outer side portion 34 easily occurs, and the effect that the base outer side portion 34 is less likely to be blocked than the base inner side portion 36 is less likely to be exerted.

On the other hand, the average density of the superabsorbent polymer particles contained in the base inner side portion 36 is preferably 0.02 to 0.35 g/cm³, more preferably 0.03 to 0.3 g/cm³, and even more preferably 0.04 to 0.25 g/cm³. In a case where the average density of the superabsorbent polymer particles contained in the base inner side portion 36 is excessively low, the superabsorbent polymer particles in the base inner side portion 36 are less likely to swell, and the main groove portion 19 easily collapses in the thickness direction T. In a case where the average density of the superabsorbent polymer particles contained in the base inner side portion 36 is excessively high, the blocking due to the swelling of the superabsorbent polymer particles in the base inner side portion 36 easily occur, and the diffusion of the bodily fluid in the lateral direction W is easily reduced.

At this time, a difference between the average density of the superabsorbent polymer particles contained in the base inner side portion 36 and the average density of the superabsorbent polymer particles contained in the base outer side portion 34 is preferably 0.01 to 0.35 g/cm³, more preferably 0.02 to 0.3 g/cm³, and even more preferably 0.03 to 0.25 g/cm³. When the difference is excessively small, blocking easily occurs in the entire base portion 20 (in a case where the average density is high), or the main groove portion 19 easily collapses in the thickness direction T (in a case where the average density is low). When the difference is excessively large, the blocking due to the swelling of the superabsorbent polymer particles in the base inner side portion 36 easily occurs.

Further, the average density of the superabsorbent polymer particles contained in the second layer 8 is preferably 0.03 to 0.4 g/cm³, more preferably 0.04 to 0.35 g/cm³, and even more preferably 0.05 to 0.3 g/cm³. In a case where the average density of the superabsorbent polymer particles contained in the second layer 8 is excessively low, the absorption amount of the bodily fluid in the entire absorbent body 7 is easily decreased. In a case where the average density of the superabsorbent polymer particles contained in the second layer 8 is excessively high, the blocking due to the swelling of the superabsorbent polymer particles in the second layer 8 easily occurs, and the absorption amount of the bodily fluid in the base portion 20 is easily decreased. It should be noted that, in the present embodiment, in the second layer 8, the average densities of the superabsorbent polymer particles in the main groove portion corresponding portion 22, the base corresponding portion 24, and the sub-groove portion corresponding portion 26 are substantially the same.

A method of measuring the average density of the superabsorbent polymer particles is as follows. Five samples having a predetermined length and a predetermined width are cut out from the absorbent core 9 (for example, 4 mm × 4 mm), the superabsorbent polymer particles contained in each sample are selected, the total mass of the superabsorbent polymer particles contained in each sample is measured, and the measurement value is divided by the volume of the sample obtained by the thickness of the sample and the area of the sample to obtain the average value.

In the present specification, in a case of evaluating the average density of the superabsorbent polymer particles in each of the portions of the base portion 20 (base outer side portion 34, base inner side portion 36) and the second layer 8 (main groove portion corresponding portion 22, base corresponding portion 24) in the absorbent core 9, the following method may be employed. For example, the absorbent article 1 serving as a sample is impregnated with liquid nitrogen so as to be frozen, and then, the absorbent article 1 is cut with a razor in the thickness direction T, so as to obtain a cross section at the surface crossing the direction in which the main groove portion 19 extends. Next, the temperature of the sample is returned to room temperature, and a cross-sectional image with a magnification of 50 times is obtained by using an electron microscope (for example, VE7800 manufactured by Keyence Corporation). In the cross-sectional image, the degrees of the average density of the superabsorbent polymer particles at each of the portions of the base portion 20, the main groove portion corresponding portion 22, and the base corresponding portion 24 is visually evaluated. It should be noted that, in a case of evaluating the degrees of the average density of the superabsorbent polymer particles in the sub-groove portion corresponding portion 26 and the average density of the superabsorbent polymer particles in each of the base portion 20 and the base corresponding portion 24, which will be described later, evaluation can be performed using a cross-sectional image in the surface crossing the direction in which the sub-groove portion 25 extends in the same method as described above, instead of the above method.

In the present embodiment, the thickness of the base outer side portion 34 in the thickness direction T is preferably in a range of 20% to 80% of the thickness of each of the plurality of base portions 20 in the thickness direction T, more preferably in a range of 30% to 70%, and even more preferably in a range of 35% to 65%. However, the thickness of the base portion 20 in the thickness direction T can be referred to as the thickness of the first layer 6 (base inner side portion 36 + base outer side portion 34) or the groove depth of the main groove portion 19.

In such an absorbent article 1, the thickness of the base outer side portion 34 is in a range of 20% to 80% of the thickness of the base portion. Therefore, even in a case where the blocking due to the swelling of the superabsorbent polymer particles in the base inner side portion 36 is likely to occur when the absorbent article 1 repeatedly absorbs the bodily fluid, the occurrence of the blocking due to the swelling of the superabsorbent polymer particles of the base outer side portion 34 can be further suppressed. That is, even when the bodily fluid is less likely to penetrate into the base portion 20 through the base inner side portion 36, the bodily fluid can be allowed to penetrate into the base portion 20 through the base outer side portion 34. Therefore, the difficulty of the bodily fluid penetrating into the base portion 20 and the difficulty of the bodily fluid diffusing in the base portion 20 in the lateral direction W can be more stably suppressed.

It should be noted that, in a case where a ratio of the thickness of the base inner side portion 36 to the thickness of the base portion 20 is excessively low, the amount of the superabsorbent polymer particles that swell in the base inner side portion 36 is decreased, and the main groove portion 19 easily collapses in the thickness direction T. On the other hand, in a case where the ratio of the thickness of the base inner side portion 36 to the thickness of the base portion 20 is excessively high, when the bodily fluid is less likely to penetrate into the base portion 20 through the base inner side portion 36 due to the swelling of the superabsorbent polymer particles, the bodily fluid that penetrates into the base portion 20 through the base outer side portion 34 is reduced, and the absorption amount of the bodily fluid is easily reduced in the base portion 20.

In the present specification, a method of measuring and calculating the relative thicknesses of the base outer side portion 34 and the base inner side portion 36 is as follows.

### (1) Acquisition of X-Ray Fluoroscopic Image of Cross Section of Sample

A sample piece including a measurement target portion is prepared from the absorbent article 1. The sample piece is an absorbent body 7 that includes the absorbent core 9 and the core wrap 11 and from which the elastic member, the liquid-permeable sheet 3, the liquid-impermeable sheet 5, and the like of the absorbent article 1 are removed, and is a test piece for X-ray cross section observation obtained by cutting the absorbent body 7 in the lateral direction to have a width of 15 mm in a plan view. It should be noted that in a case where the absorbent body 7, the liquid-permeable sheet 3, and the liquid-impermeable sheet 5 are integrally formed, and in a case where the liquid-permeable sheet 3 and the liquid-impermeable sheet 5 cannot be removed without destroying the shape of the absorbent body 7, the sample piece is a test piece for X-ray cross section observation having the absorbent body 7 that includes the absorbent core 9 and the core wrap 11, the liquid-permeable sheet 3, and the liquid-impermeable sheet 5 of the absorbent article 1 from which the elastic member is removed, and obtained by cutting the absorbent body 7 in the lateral direction to a width of 15 mm in a plan view. Next, by using the following X-ray photographing apparatus and image processing software, a cross section of the sample piece is photographed to obtain an internal cross-sectional photograph including the thickness direction T thereof in BMP format. Then, the obtained X-ray fluoroscopic image having a field size of 30 × 20 mm is tiled in the lateral direction of the absorbent body 7, and the thickness of the absorbent body 7, the thickness of the base outer side portion 34, and the groove depth of the main groove portion 19 are calculated using the number of pixels of the composite image analysis software. Thereafter, assuming that the total thickness of the absorbent body 7 is 100, the ratio of the groove depth of the main groove portion 19 and the thickness of the base outer side portion 34 is calculated. Measurement and photographing apparatus: X-ray fluoroscopy apparatus: FLEX-M863 (manufactured by Beam Sense Co., Ltd.)
Photographing conditions: tube voltage 35 kV, tube current 100 µA, pixel size: 20 × 20 µm Number of pixels: 1500 × 1000 pixels, Resolution: 2 µm (geometric magnification: 10)
Image connection software/image measurement software: UN Analyzer (manufactured by Unique Co., Ltd.)

### (2) Method of Measuring and Calculating Relative Thickness And Depth of Measurement Target Portion in Sample

From the internal cross-sectional photograph of the BMP format obtained in (1), the number of pixels corresponding to the total thickness of the absorbent body 7, the thickness of the base outer side portion 34, and the groove depth of the main groove portion 19 are read. The total thickness of the absorbent body 7, the groove depth of the main groove portion 19, the thickness of the base outer side portion 34, the thickness of the base inner side portion 36 (the groove depth of the main groove portion 19 - the thickness of the base outer side portion 34), and the thickness of the second layer 8 (the total thickness of the absorbent body 7 - the groove depth of the main groove portion 19) are calculated. Thereafter, assuming that the total thickness of the absorbent body 7 is 100, the ratios of the groove depth of the main groove portion 19, the thickness of the base outer side portion 34, the thickness of the base inner side portion 36, and the thickness of the second layer 8 are calculated as the relative thickness and depth.

In a preferable aspect of the present embodiment, in the base portion 20, at one end portion and the other end portion, and at the central part between the one end portion and the other end portion in the lateral direction W, the average density of the superabsorbent polymer particles contained in each of the one end portion and the other end portion is lower than the average density of the superabsorbent polymer particles contained in the central part. However, the end portion refers to a range of 1/5 of the length of the base portion 20 in the lateral direction W from the end edge of the base portion 20 in the lateral direction W. In such an absorbent article 1, the swelling of two end portions that come into contact with the main groove portions 19 in the base portion can be suppressed, and the inhibition of the bodily fluid drawing action can be suppressed. Therefore, the absorption performance in a case where the bodily fluid is repeatedly absorbed in the absorbent article 1 can be enhanced.

### (Second embodiment)

Hereinafter, an absorbent article according to an embodiment will be described.

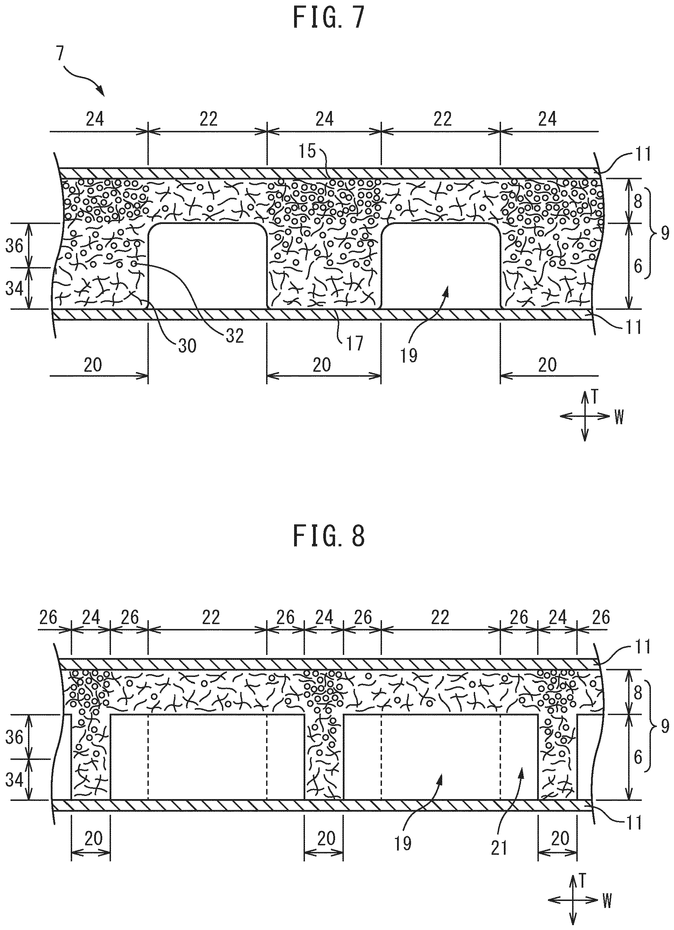

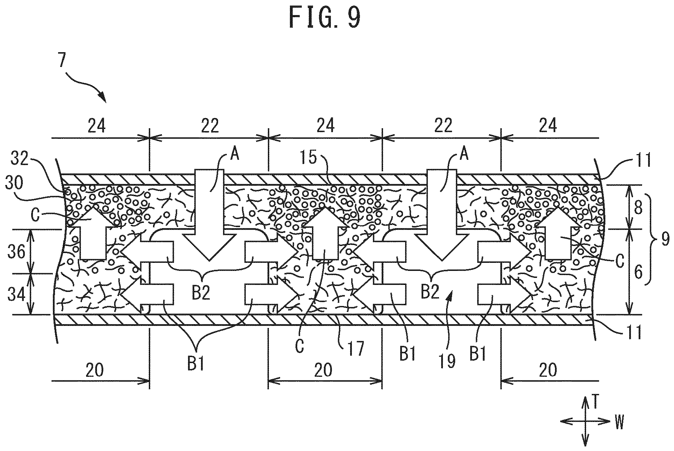

In the present embodiment, differences from the first embodiment will be mainly described. FIGS. 7 to 9 are view showing configuration examples of the absorbent article 1 according to the embodiment, specifically, a disposable diaper. Specifically, FIGS. 7 and 8 are a cross-sectional view taken along the line IV-IV in FIG. 2 and a cross-sectional view taken along the line V-V, respectively. FIG. 9 is a schematic view showing the flow of the bodily fluid in the cross-section taken along the line IV-IV in FIG. 2.

As shown in FIG. 7, the absorbent core 9 has different average densities of the superabsorbent polymer particles in the base outer side portion 34 and the base inner side portion 36 of the base portion 20, the main groove portion corresponding portion 22, and the base corresponding portion 24. Specifically, the average density of the superabsorbent polymer particles contained in the base portion 20 (the base outer side portion 34 and the base inner side portion 36) and the average density of the superabsorbent polymer particles contained in the main groove portion corresponding portion 22 are lower than the average density of the superabsorbent polymer particles contained in the base corresponding portion 24.

In the present embodiment, the average densities of the superabsorbent polymer particles contained in the base outer side portion 34 and the base inner side portion 36 of the base portion 20 are as described in the first embodiment. The average density of the superabsorbent polymer particles contained in the main groove portion corresponding portion 22 are preferably 0 to 0.25 g/cm³, more preferably 0 to 0.2 g/cm³, and even more preferably 0 to 0.15 g/cm³. On the other hand, the average density of the superabsorbent polymer particles contained in the base corresponding portion 24 is preferably 0.1 to 0.45 g/cm³, more preferably 0.1 to 0.4 g/cm³, and even more preferably 0.1 to 0.35 g/cm³.

As described above, in the present embodiment, the average densities of the superabsorbent polymer particles have a relationship of the base portion 20 (base outer side portion 34 and base inner side portion 36) and the main groove portion corresponding portion 22 < the base corresponding portion 24. In such a configuration, in the present embodiment, as shown in FIG. 9, the bodily fluid moves in the absorbent core 9 in substantially the following order.
(i) The bodily fluid that has reached the core wrap 11 permeates into the main groove portion corresponding portion 22 in the second layer 8, where the average density of the superabsorbent polymer particles is relatively low and the bodily fluid easily penetrates into the main groove portion corresponding portion 22, and further moves to the main groove portion 19 by gravity (arrow A).
(ii) The bodily fluid that has moved to the main groove portion 19 diffuses in the longitudinal direction L while exuding into the base outer side portion 34 and the base inner side portion 36 of the base portion 20 that are adjacent in the lateral direction W (arrows B1 and B2).
(iii) The bodily fluid that cannot be held in the base portion 20 is sucked up by the base corresponding portion 24 having the highest average density of the superabsorbent polymer particles in the absorbent core 9, and is held therein (arrow C).

In the absorbent article 1, both the average density of the superabsorbent polymer particles contained in the base portion 20 and the average density of the superabsorbent polymer particles contained in the main groove portion corresponding portion 22 are lower than the average density of the superabsorbent polymer particles contained in the base corresponding portion 24. Therefore, when the bodily fluid is absorbed, the bodily fluid can easily permeate into the main groove portion 19 through the main groove portion corresponding portion 22, the water flow function of the main groove portion 19 can be secured, and the bodily fluid that cannot be held in the base portion 20 can be easily absorbed in the base corresponding portion 24. As described above, in the absorbent article 1, the bodily fluid can be absorbed by a bodily fluid absorption cycle in which the bodily fluid permeates from the main groove portion corresponding portion 22 to the main groove portion 19 (also referred to as "bodily fluid permeation action"), the permeated bodily fluid is drawn from the main groove portion 19 to the adjacent base portion 20 (also referred to as "bodily fluid drawing action"), and when the drawn bodily fluid cannot be held in the base portion 20, the bodily fluid is sucked up by the base corresponding portion 24 (also referred to as "bodily fluid sucking action"). At this time, in the absorbent article 1, even when blocking in the base inner side portion 36 is likely to occur, the occurrence of blocking due to the swelling of the superabsorbent polymer particles in the base outer side portion 34 can be suppressed, and thus the bodily fluid absorption cycle can be stably maintained. Therefore, the absorption performance in a case where the bodily fluid is repeatedly absorbed in the present absorbent article 1 can be enhanced.

In a preferable aspect of the present embodiment, the average density of the superabsorbent polymer particles contained in the main groove portion corresponding portion 22 is lower than the average density of the superabsorbent polymer particles contained in the base inner side portion 36 of the base portion 20. In such an absorbent article 1, the swelling of the main groove portion corresponding portion 22 can be suppressed, and the inhibition of the bodily fluid permeation action can be suppressed. Therefore, the absorption performance in a case where the bodily fluid is repeatedly absorbed in the absorbent article 1 can be enhanced.

In a preferable aspect of the present embodiment, the average density of the superabsorbent polymer particles contained in the base outer side portion 34 of the base portion 20 is lower than the average density of the superabsorbent polymer particles contained in the main groove portion corresponding portion 22. In such an absorbent article 1, the swelling of the base outer side portion 34 can be suppressed and the inhibition of the bodily fluid drawing action can be suppressed. Therefore, the absorption performance in a case where the bodily fluid is repeatedly absorbed in the absorbent article 1 can be enhanced.

In a preferable aspect of the present embodiment, the average density of the superabsorbent polymer particles contained in the sub-groove portion corresponding portion 26 is lower than the average density of the superabsorbent polymer particles contained in the base corresponding portion 24. At this time, the average density of the superabsorbent polymer particles contained in the sub-groove portion corresponding portion 26 is approximately the same as the average density of the superabsorbent polymer particles contained in the main groove portion corresponding portion 22. In such an absorbent article 1, when the bodily fluid is absorbed, the bodily fluid can easily permeate into the sub-groove portion 21 through the sub-groove portion corresponding portion 26, and the water flow function of the sub-groove portion 21 can be secured. In addition, the bodily fluid can be further absorbed by a bodily fluid absorption cycle in which while being diffused in a direction crossing the longitudinal direction L along the sub-groove portion 21, the bodily fluid is drawn into the base portion 20 (base outer side portion 34, base inner side portion 36) and held therein, and the bodily fluid that cannot be held in the base portion 20 is sucked up to the base corresponding portion 24. Therefore, the absorption performance in a case where the bodily fluid is repeatedly absorbed in the absorbent article 1 can be enhanced.

In a preferable aspect of the first and second embodiments, as shown in FIG. 2, the absorbent body 7 is partitioned into a right region RA and a left region LA by a longitudinal central axis line VL that bisects the absorbent body 7 in the lateral direction W. Further, the absorbent body 7 includes two main groove portions 19 in each of the right region RA and the left region LA.

In the first and second embodiment, as shown in FIG. 1, the absorbent article 1 is partitioned in the longitudinal direction L into three regions: a front waist region FW, a back waist region RW, and a crotch region C between the front waist region FW and the back waist region RW. Further, the absorbent body 7 is arranged across the three regions, and the sub-groove portions 21 are provided in all of the three regions.

In the first and second embodiments, the groove portion 18 is not limited to the configuration in which the main groove portion 19 linearly extends along the longitudinal direction L and the sub-groove portion 21 is orthogonal to the main groove portion 19. For example, the groove portion 18 may include only the main groove portion 19 and may not include the sub-groove portion 21. Further, the main groove portion 19 may not extend across the front waist region FW, the crotch region C, and the back waist region RW in the longitudinal direction L of the absorbent core 9, and may be arranged only in the region of the crotch region C, for example. Further, each of the plurality of main groove portions 19 may be arranged so as to extend in a zigzag shape along the longitudinal direction L in a plan view, and to alternately form a broad base portion having a relatively large width and a narrow base portion having a relatively narrow width with the main groove portions 19 which are adjacent to each other in the longitudinal direction L. Alternatively, each of the plurality of main groove portions may extend in a zigzag shape along the longitudinal direction L in a plan view, and the adjacent main groove portions 19 may be arranged in a manner such that the width of the base portion 20 becomes constant, or each of the plurality of main groove portions 19 may extend in a wavy shape in a plan view.

In the first and second embodiments, as shown in FIG. 4, the core wrap 11 that covers the non-skin facing surface 17 of the absorbent core 9 may be arranged so as to protrude toward the skin facing surface 15 side although not to come into contact with the main groove portion corresponding portion 22 in the main groove portion 19. In such a case, the diffusibility of the bodily fluid in the longitudinal direction L and the lateral direction W in the main groove portion 19 can be enhanced. Alternatively, the core wrap 11 that covers the non-skin facing surface 17 of the absorbent core 9 may come into contact with the main groove portion corresponding portion 22, or may be partially or continuously joined to the main groove portion corresponding portion 22 with a compressed portion or a joining portion. As described above, the main groove portion 19 is formed by intermittently compressing or joining a part of the width of the main groove portion corresponding portion 22 along the main groove portion 19, and thus the wearer is less likely to feel the hardness of the compressed portion or the joining portion. In addition, since the main groove portion 19 is formed by continuously compressing or joining a part of the width of the main groove portion corresponding portion 22 in the longitudinal direction L, the absorbent article 1 is easily folded with respect to the main groove portion 19, and exhibits excellent fitting property.

In the first and second embodiment, in the absorbent article 1, the main groove portion 19 has a width of preferably 0.5 to 3.0 times, more preferably 0.8 to 2.5 times, and even more preferably 1.0 to 2.0 times as much of the thickness of the absorbent body 7. When the width of the main groove portion 19 is within the above range, the main groove portion 19 easily maintains its water flow function after the absorbent article 1 absorbs the bodily fluid.

In the present specification, unless otherwise specified, the thickness (mm) of an object (for example, an absorbent body or an absorbent core) is measured as follows. FS-60DS [the measurement surface: 44 mm (in diameter), the measurement pressure: 3 g/cm²] manufactured by Daiei Kagaku Seiki MFG, Co., Ltd. is prepared, five different portions of the object are applied with pressure under the standard condition (the temperature: 23±2°C, the relative humidity: 50±5%), the thickness at each of the portions 10 seconds after being applied with pressure is measured, and the average value of the 5 measurement values is used as the thickness of the object.

In the first and second embodiments, a space between the sub-groove portions 21 that are adjacent to each other in the lateral direction W is preferably 10% to 80%, and more preferably 20% to 60% of the space between the plurality of main groove portions 19 that are adjacent to each other in the lateral direction W. As described above, when the sub-groove portions 21 have a predetermined space, a predetermined width can be secured in the base portion 20 present between the sub-groove portions 21, the bodily fluid absorbed in the base portion 20 through the sub-groove portions 21 is easily diffused in the longitudinal direction L, and even when the bodily fluid is repeatedly absorbed, the absorbent article 1 has excellent diffusibility, absorption rate, and liquid return suppression.

### <Method of Manufacturing Absorbent Article>

In a case of manufacturing the absorbent article 1 having the configuration described in the above embodiment, there is no limitation on the manufacturing method. However, for example, the following method can be used. Incidentally, in the present specification, the "the machine direction of a material or a product" is referred to as "the MD direction", "the direction orthogonal to the MD direction on a horizontal plane" (that is, the width direction of the manufacturing line) is referred to as "the CD direction", and "the direction orthogonal to the MD direction and the CD direction" (that is, the vertical direction of the manufacturing line) is referred to as "the TD direction" .

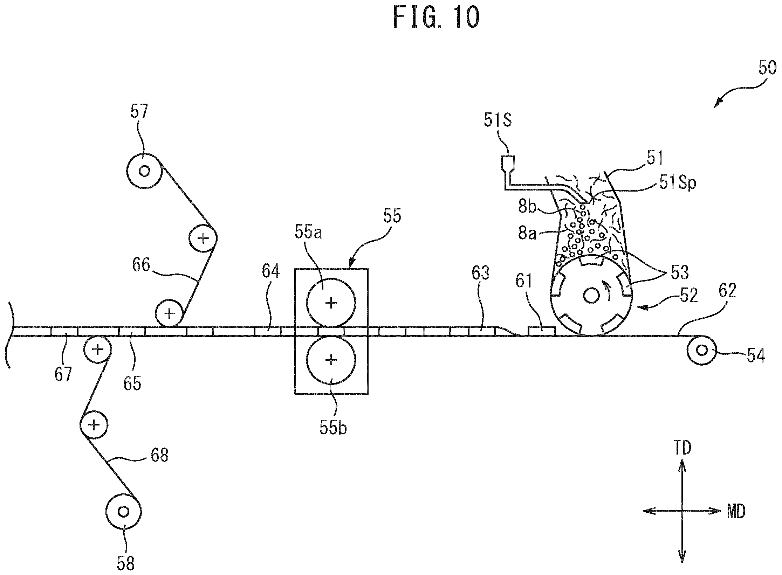

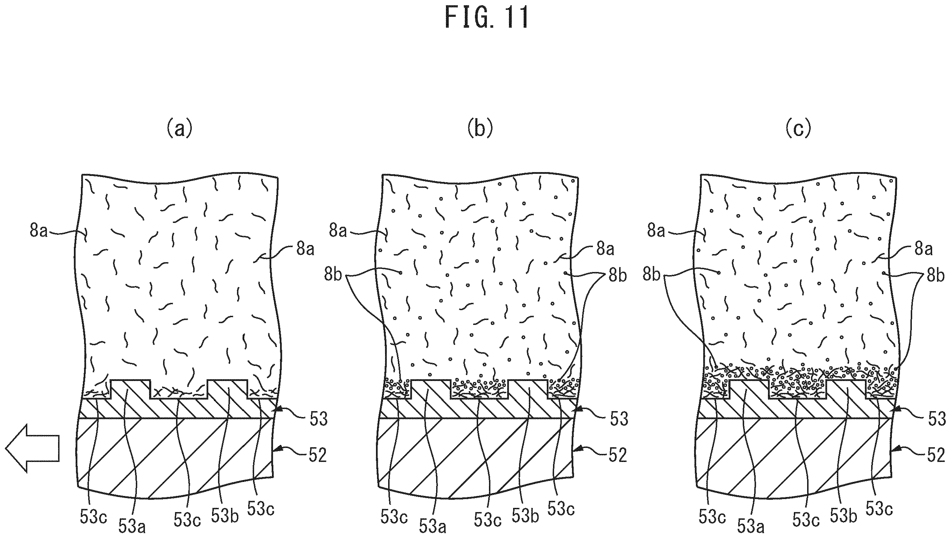

FIG. 10 is a schematic view showing a configuration example of a manufacturing apparatus 50 of manufacturing the absorbent article 1 according to the embodiment. In addition, FIGS. 11(a) to 11(c) are schematic views showing the state in which the absorbent material is supplied on a suction drum 52 of the manufacturing apparatus 50 in FIG. 10.

The manufacturing apparatus 50 includes a transport duct 51 and a suction drum 52. The transport duct 51 transports the absorbent material that includes an opened water-absorbent fibers 8a and superabsorbent polymer particles 8b to the suction drum 52. The transport duct 51 includes a transport duct nozzle 51S that discharges the superabsorbent polymer particles 8b from a discharge port 51Sp to the suction drum 52. The suction drum 52 is rotatable, and sucks the absorbent material in the transport duct 51 and laminates the absorbent material on a plurality of concave mold members 53 arranged with a certain space along the circumferential direction of the outer circumferential surface to form a first laminated body 61 which becomes the absorbent core 9 of the absorbent body 7 in the later process.

The manufacturing apparatus 50 further includes an unwinding roll 54 for the core wrap continuous body. The unwinding roll 54 unwinds a long core wrap continuous bodies 62 toward the suction drum 52. The suction drum 52 places the first laminated body 61 on the outer circumferential surface of the suction drum on the core wrap continuous body 62. The first laminated body 61 placed on the core wrap continuous body 62 is covered with the core wrap continuous body 62 to form a second laminated body 63. The manufacturing apparatus 50 further includes a pressing device 55. The pressing device 55 includes a pair of press rolls 55a and 55b that apply pressure and compress the second laminated body 63 in the thickness direction (TD direction). A third laminated body 64 is formed by pressing the second laminated body 63. The manufacturing apparatus 50 further includes an unwinding roll 57 for a liquid-permeable sheet continuous body. The unwinding roll 57 unwinds and laminates a long liquid-permeable sheet continuous body 66 that serves as the liquid-permeable sheet 3 on one surface (in the case of FIG. 10, the upper surface) of the third laminated body 64. The liquid-permeable-sheet continuous body 66 is laminated on the third laminated body 64 to form a fourth laminated body 65. The manufacturing apparatus 50 further includes an unwinding roll 58 for a liquid-impermeable sheet continuous body. The unwinding roll 58 unwinds and joins a long liquid-impermeable sheet continuous body 68 that serves as the liquid-impermeable sheet 5 to the surface (in the case of FIG. 10, the lower surface) of the fourth laminated body 65 opposite to the liquid-permeable sheet continuous body 66. The liquid-impermeable sheet continuous body 68 is laminated on the fourth laminated body 65 to form a fifth laminated body 67.

It should be noted that the manufacturing apparatus 50 includes a device (not shown) that cuts the fifth laminated body 67 into the shape of the absorbent article 1 as a product so as to be a single absorbent article 1, and each of the devices that compresses leakproof walls 101 and the tape fasteners 109 onto the fifth laminated body 67 on the downstream side in the MD direction with respect to the unwinding roll 58. However, since these devices are ordinary devices known in this technical field, the detailed explanation is omitted.

In a case where the absorbent article 1 is manufactured by using the manufacturing apparatus 50 described above, the following steps are performed. That is, the following steps are sequentially performed: a first step of forming the first laminated body 61; a second step of covering the first laminated body 61 with the core wrap continuous body 62 to form the second laminated body 63; and a third step of compressing the second laminated body 63 in the TD direction with the pressing device 55 to form the third laminated body 64. Further, a fourth step of laminating the liquid-permeable sheet continuous body 66 on the third laminated body 64 to form the fourth laminated body 65, and a fifth step of joining the liquid-impermeable sheet continuous body 68 to the fourth laminated body 65 to form the fifth laminated body 67 are sequentially performed.

First, the first step of forming the first laminated body 61 that will ultimately constitute the absorbent core 9 of the absorbent body 7 is performed. In the first step, the absorbent material which includes the water-absorbent fibers 8a and the superabsorbent polymer particles 8b is sucked by the suction drum 52 through the transport duct 51, and the absorbent material is laminated in the mold member 53 on the outer circumferential surface of the suction drum 52 to form the first laminated body 61.

Here, the mold member 53 includes four pairs of protruding portions (in FIG. 11, only 53a and 53b are shown) having a quadrangular cross section which extend in the circumferential direction of the suction drum 52 in a bottom portion 53c. Specifically, each of the protruding portions 53a and 53b has a rectangular shape. These protruding portions 53a and 53b are arranged at positions adapted to the positions of the main groove portions 19 of the absorbent body 7 so as to have the shape and the width in the longitudinal direction adapted to the shape and the width in the longitudinal direction of the main groove portions 19.

FIG. 11(a) shows the state in which the absorbent material is supplied on the suction drum 52 in the first region (for example, the first 1/5 of the entire region) of the region in which the absorbent material is supplied in the transport duct 51. This region is a region controlled so that the superabsorbent polymer particles 8b discharged from the discharge port 51Sp are supplied by a small amount or almost no amount of the superabsorbent polymer particles is supplied and the water-absorbent fibers 8a are supplied by a predetermined amount. Therefore, the water-absorbent fibers 8a are mainly supplied to and laminated on the bottom portion 53c of the mold member 53 of the suction drum 52. The total laminated thickness of the superabsorbent polymer particles 8b and the water-absorbent fibers 8a is smaller than the height of the protruding portions 53a and 53b. As a result, portions corresponding to the base outer side portions 34 are formed.

FIG. 11(b) shows the state in which the absorbent material is supplied on the suction drum 52 in an intermediate region (for example, 1/5 of the middle area of the entire region) of the region in which the absorbent material is supplied in the transport duct 51. This region is a region controlled so that the superabsorbent polymer particles 8b discharged from the discharge port 51Sp are supplied by another predetermined amount (more than the "small amount" of (a)) and the water-absorbent fibers 8a are supplied by a predetermined amount. Therefore, the superabsorbent polymer particles 8b and the water-absorbent fibers 8a are supplied and laminated on the upper part of the bottom portions 53c and the protruding portions 53a and 53b of the mold member 53 on the outer circumferential surface of the suction drum 52. The total laminate thickness of the superabsorbent polymer particles 8b and the water-absorbent fibers 8a is larger than the height of the protruding portions 53a and 53b. Accordingly, portions corresponding to the base inner side portions 36 and a part of the second layer 8 are further formed.

FIG. 11(c) shows the state in which the absorbent material is supplied on the suction drum 52 in the last region (for example, the last 1/5 of the entire region) of the region in which the absorbent material is supplied in the transport duct 51. This region is a region controlled so that of the superabsorbent polymer particles 8b discharged from the discharge port 51Sp are supplied by small amount or another predetermined amount and the water-absorbent fibers 8a are supplied by a predetermined amount. Therefore, the superabsorbent polymer particles 8b and the water-absorbent fibers 8a are supplied and laminated on the upper part of the bottom portions 53c and the protruding portions 53a and 53b of the mold member 53 of the suction drum 52. The total laminate thickness of the superabsorbent polymer particles 8b and the water-absorbent fibers 8a is larger than the height of the protruding portions 53a and 53b. As a result, the remainder of the second layer 8 is further formed, and finally the absorbent core 9 is formed.

It should be noted that, in a case where it is desired to decrease the average density of the superabsorbent polymer particles in the main groove portion corresponding portion 22 (or the sub-groove portion corresponding portion 26) of the second layer 8 in the second embodiment, the manufacturing method of Patent Literature 1 (Japanese Unexamined Patent Publication No. 2018-872) can be applied. For example, when a transport duct nozzle 51H that discharges the superabsorbent polymer particles 8b in a relatively large amount is further arranged in the transport duct 51 and the second layer 8 is formed, the absorbent material is supplied from the transport duct nozzle 51H present at a lower position than the transport duct nozzle 51S. In such a case, since the superabsorbent polymer particles 8b are contained in a large discharge amount, when the superabsorbent polymer particles 8b collide with the protruding portions 53a and 53b, the superabsorbent polymer particles 8b are easily bounced back, and due to that, in the first laminated body 61, there is a portion in which the average density of the superabsorbent polymer particles 8b is low in the portions on the protruding portions 53a and 53b. The portions on the protruding portions 53a and 53b serve as portions corresponding to the main groove portion corresponding portions 22 (or the sub-groove portion corresponding portions 26).

In the second step, the rotated suction drum 52 transfers the first laminated body 61 in the mold member 53 onto the core wrap continuous body 62 (coated with an adhesive) that is unwound from the unwinding roll 54 for the core wrap continuous body and is moved in the MD direction. Then, the core wrap continuous body 62 is folded along the outer circumferential surface of the first laminated body 61 in the CD direction orthogonal to the MD direction with folding means (not shown), and the core wrap continuous body 62 is covered with the first laminated body 61 by being wound to form the long second laminated body 63. Next, in the third step, the second laminated body 63 is passed between the pair of press rolls 55a and 55b of the pressing device 55 to compress the second laminated body 63 in the TD direction. At this time, the third laminated body 64 is formed. Next, in the fourth step, the liquid-permeable continuous body 66 which is unwound from the unwinding roll 57 for the liquid-permeable continuous body is laminated on the upper surface of the third laminated body 64 with an adhesive agent such as a hot-melt adhesive agent to form the long fourth laminated body 65. Next, in the fifth step, the liquid-impermeable sheet continuous body 68 which is unwound from the unwinding roll 58 for the liquid-impermeable sheet continuous body is joined to the lower surface of the fourth laminated body 65 with an adhesive such as a hot-melt adhesive to form the long fifth laminated body 67. After the fifth step is completed, the fifth laminated body 67 is cut into the shape of the absorbent article 1 by a cutting device. Due to that, the absorbent article 1 is completed.

In the above manufacturing method, an aspect has been described in which the mold member 53 is provided with the protruding portions 53a and 53b for forming the main groove portion 19. However, as a method of manufacturing the absorbent article, the mold member 53 may be provided with the protruding portions for forming the sub-groove portions 21. Further, each step can be appropriately changed without departing from the scope of the present disclosure.

### Examples

Hereinafter, the present invention will be described by showing examples, but the present invention is not limited to these examples.

### (a) Sample

An absorbent core including pulp (average basis weight: 220 g/m²) and the superabsorbent polymer particles (average basis weight: 230 g/m²) and having a size of 350 mm x 120 mm (longitudinal direction x lateral direction) was manufactured according to the above manufacturing method. However, in the above manufacturing method, in the step of FIG. 11(a), the absorbent body was substantially formed of only the water-absorbent fibers, and in the steps of FIG. 11(b) and FIG. 11(c), the absorbent body was substantially uniformly formed of the water-absorbent fibers and the superabsorbent polymer particles. In the absorbent cores of Examples 1 to 4, the amount of the water-absorbent fibers supplied to the step of FIG. 11(a) was set to be 7.5%, 15%, 22.5%, and 30% of the total amount of the supplied water-absorbent fibers. The basis weights generally corresponded to 17 g/m², 33 g/m², 50 g/m², and 66 g/m². On the other hand, in the absorbent cores of Comparative Examples 1 and 2, the amount of the water-absorbent fibers supplied to the step of FIG. 11(a) was set to 0% and 50% of the total amount of the supplied water-absorbent fibers. The basis weight generally corresponded to 0, 110 g/m². In the absorbent core, four main groove portions (width: 4 mm) were arranged in the first layer with a space of 18 mm.

Next, each of the absorbent cores was covered with two sheets of tissue (basis weight: 16 g/m ², 400 mm × 150 mm) as a core wrap with a hot-melt adhesive interposed therebetween. The thickness of the absorbent core was adjusted by pressing the absorbent core covered with two pieces of tissue with a hydraulic press machine. Thus, the absorbent bodies of Examples 1 to 4 were formed from the absorbent cores of Examples 1 to 4, and the absorbent bodies of Comparative Examples 1 and 2 were formed from the absorbent cores of Comparative Examples 1 and 2. Thereafter, a liquid-permeable sheet (air-through nonwoven fabric) was attached to the second layer side of each absorbent body, and a liquid-impermeable sheet (polyethylene film) was attached to the first layer side. Accordingly, simple absorbent articles of Examples 1 to 4 were formed from the absorbent bodies of Examples 1 to 4, and simple absorbent articles of Comparative Examples 1 and 2 were formed from the absorbent bodies of Comparative Examples 1 and 2.

### (B) Evaluation

The absorbent articles of Examples 1 to 4 and the absorbent articles of Comparative Examples 1 and 2 were subjected to the following absorbent test, and the absorption rate and liquid return amount (rewet) were evaluated. The results of the evaluation are shown in Table 1.

### [Absorbent Test]

### (1) The absorbent article is set in a U-shaped instrument having a substantially U-shaped side view.

It should be noted that the absorbent article is set so that the central position of the absorbent body in the longitudinal direction matches the central part of the U-shaped instrument (the position at which the height is the lowest).

### <First Cycle>

(2) 80 mL of artificial urine (first time) is injected from a burette at a rate of 80 mL/10 sec to the central position of the absorbent body.
(3) The time from the start of injection of the artificial urine of the first time until the artificial urine within the U-shaped instrument disappears is recorded as the absorption time (80 mL).
(4) After 3 minutes from the start of injection of the artificial urine of the first time, in the liquid-permeable sheet of the absorbent article, the contour (80 mL) of the region in which the artificial urine has diffused is recorded.

### <Second Cycle>

(5) After 10 minutes from the start of the injection of the artificial urine of the first time, 80 mL of artificial urine (second time) is injected into the central position of the absorbent body from a burette at a rate of 80 mL/10 sec.
(6) The time from the start of injection of the artificial urine of the second time until the artificial urine within the U-shaped instrument disappears is recorded as the absorption time (160 mL).
(7) After 3 minutes from the start of injection of the artificial urine of the second time, in the liquid-permeable sheet of the absorbent article, the contour (160 mL) of the region in which the artificial urine has diffused is recorded.

### <Third Cycle>

(8) The operations of (5) and (7) are repeated, the absorption time (240 mL) is measured and the contour (240 mL) is recorded ("U-shaped three cycle absorption rate (s/80 ml)" in Table 1).
(9) After 4 minutes from the start of the injection of the artificial urine of the third time, the absorbent article is removed from the U-shaped instrument, and the absorbent article is expanded on an acrylic flat plate in a manner such that the liquid-permeable sheet is to be the upper surface, and is left to stand for 1 minute.
(10) After 5 minutes from the start of the injection of the artificial urine of the third time, approximately 60 g of filter paper with a size of 100 mm × 100 mm is placed on the liquid-permeable sheet of the absorbent article with the artificial urine injection point as the center. Further, a weight of 3.5 kg with a size of 100 mm × 100 mm × 50 mm (height) is placed thereon. It should be noted that as for the filter paper, the mass before the test is measured in advance.
(11) After 8 minutes from the start of injection of the artificial urine of the third time, the weight is removed, the mass of the filter paper is measured, the mass of the filter paper before the test is subtracted, and the difference is regarded as the liquid return amount ("U-shaped three cycle liquid return amount (g/240 ml) "in Table 1).

It should be noted that the artificial urine was prepared by dissolving 200 g of urea, 80 g of sodium chloride, 8 g of magnesium sulfate, 3 g of calcium chloride, and approximately 1 g of a dye (blue No.1) in 10 L of ion exchange water.

**[Table 1]**

| | Thickness of base outer side portion/thickness of base portion (%) | U-shaped three cycle absorption rate (s/80 ml) | U-shaped three cycle liquid return amount (g/240 ml) | Evaluation |
|---|---|---|---|---|
| Example 1 | 23 | 25 | 36 | B |
| Example 2 | 39 | 22 | 31 | A |
| Example 3 | 60 | 20 | 29 | A |
| Example 4 | 76 | 25 | 34 | B |
| Comparative Example 1 | 13 | 26 | 37 | C |
| Comparative Example 2 | 93 | 30 | 30 | C |

Here, in the evaluation, A represents suitable (more satisfactory), B represents suitable, and C represents unsuitable.

Compared with the absorbent articles of Comparative Examples 1 and 2, the absorbent articles of Examples 1 to 4, which completed the three cycle absorbent test simultaneously satisfied both standards of an absorption time of 25 s/80 ml or shorter and a liquid return amount of 36 g/240 ml or less. In particular, in Examples 2 and 3, the absorption time was 22 s/80 ml or shorter, and the liquid return amount was 31 g/240 ml or less, which were very satisfactory. Accordingly, it was found that the ratio of the thickness of the base outer side portion to the thickness of the base portion is preferably 20% to 80% and more preferably 30% to 70%.

Examples 1 to 4 are reference examples not falling under the scope of the present invention.

The present invention is not limited to each of the above-described embodiments and can be appropriately combined, modified, or the like without departing from the scope of the present invention.

### REFERENCE SIGNS LIST

1: absorbent article
6: First layer
8: Second layer
9: absorbent core
18: Groove portion
19: Main groove portion
20: Base portion
34: Base outer side portion
36: Base inner side portion

## Claims

1. An absorbent article having a longitudinal direction, a lateral direction, and a thickness direction, the absorbent article comprising:
an absorbent core (9) including superabsorbent polymer particles,
wherein the absorbent core (9) has a non-skin facing surface, and a skin facing surface, and is formed of a first layer (6) on a non-skin facing surface side, and a second layer (8) on a skin facing surface side,
the first layer (6) includes:
a plurality of groove portions (18) that includes a plurality of main groove portions (19) extending in the longitudinal direction and penetrating the first layer (6) in the thickness direction, and
a plurality of base portions (20) that extend in the longitudinal direction,
each of the plurality of main groove portions (19) and each of the plurality of base portions (20) are arranged alternately in the lateral direction,
each of the plurality of base portions (20) is formed of:
a base inner side portion (36) adjacent to the non-skin facing surface side of the second layer (8), and
a base outer side portion (34) adjacent to the non-skin facing surface side of the base inner side portion (36), and
an average density of the superabsorbent polymer particles contained in the base outer side portion (34) is lower than an average density of the superabsorbent polymer particles contained in the base inner side portion (36)
wherein the second layer (8) includes:
a plurality of main groove portion corresponding portions (22) and a plurality of base corresponding portions (24) arranged at positions overlapping with the plurality of main groove portions (19) and the plurality of base portions (20) in the thickness direction, respectively, and charactertised in that:
both an average density of the superabsorbent polymer particles contained in each of the plurality of base portions (20) and an average density of the superabsorbent polymer particles contained in each of the plurality of main groove portion corresponding portions (22) are lower than an average density of the superabsorbent polymer particles contained in each of the plurality of base corresponding portions (24).

2. The absorbent article according to claim 1,
wherein a thickness of the base outer side portion (34) in the thickness direction is in a range of 20% to 80% of a thickness of each of the plurality of base portions (20) in the thickness direction.

3. The absorbent article according to claim1 or 2,
wherein the average density of the superabsorbent polymer particles contained in each of the plurality of main groove portion corresponding portions (22) is lower than the average density of the superabsorbent polymer particles contained in the base inner side portion (36) of each of the plurality of base portions (20).

4. The absorbent article according to claim 3,
wherein the average density of the superabsorbent polymer particles contained in the base outer side portion (34) of each of the plurality of base portions (20) is lower than the average density of the superabsorbent polymer particles contained in each of the plurality of main groove portion corresponding portions (22).

5. The absorbent article according to any one of claims 1 to 4,
wherein each of the plurality of groove portions (18) further includes sub-groove portions (21) that penetrate the first layer (6) in the thickness direction and are present in communication with each of the plurality of main groove portions (19) through a base end with a predetermined space in a direction crossing each of the plurality of main groove portions (19).

6. The absorbent article according to claim 5,
wherein the second layer (8) further includes:
sub-groove portion corresponding portions (26) arranged at positions overlapping with the sub-groove portions (21) in the thickness direction, and
an average density of the superabsorbent polymer particles contained in the sub-groove portion corresponding portion (26) is lower than an average density of the superabsorbent polymer particles contained in each of the plurality of base corresponding portions (24).

7. The absorbent article according to any one of claims 1 to 6,
wherein in each of the plurality of base portions (20), at one end portion and the other end portion in the lateral direction, and at a central part between the one end portion and the other end portion,
an average density of the superabsorbent polymer particles contained in each of the one end portion and the other end portion is lower than an average density of the superabsorbent polymer particles contained in the central part.

## Patentansprüche

1. Absorbierender Artikel, der eine Längsrichtung, eine laterale Richtung und eine Dickenrichtung aufweist, wobei der absorbierende Artikel Folgendes umfasst:
einen Saugkern (9), der superabsorbierende Polymerpartikel einschließt,
wobei der Saugkern (9) eine der Haut nicht zugewandte Oberfläche und eine der Haut zugewandte Oberfläche aufweist und aus einer ersten Schicht (6) auf einer der Haut nicht zugewandten Oberflächenseite und einer zweiten Schicht (8) auf einer der Haut zugewandten Oberflächenseite gebildet ist,
wobei die erste Schicht (6) Folgendes einschließt:
eine Vielzahl von Rillenteilen (18), die eine Vielzahl von Hauptrillenteilen (19) einschließt, sich in der Längsrichtung erstrecken und die erste Schicht (6) in der Dickenrichtung penetrieren, und
eine Vielzahl von Basisteilen (20), die sich in der Längsrichtung erstrecken,
wobei jeder der Vielzahl von Hauptrillenteilen (19) und jeder der Vielzahl von Basisteilen (20) abwechselnd in der lateralen Richtung angeordnet sind,
wobei jeder der Vielzahl von Basisteilen (20) aus Folgenden gebildet ist:
einem Innenseitenbasisteil (36) angrenzend an die der Haut nicht zugewandten Oberflächenseite der zweiten Schicht (8) und
einen Außenseitenbasisteil (34) angrenzend an die der Haut nicht zugewandten Oberflächenseite des Innenseitenbasisteils (36), und
wobei eine durchschnittliche Dichte der superabsorbierenden Polymerpartikel, die in dem Außenseitenbasisteil (34) enthalten sind, geringer ist als eine durchschnittliche Dichte der superabsorbierenden Polymerpartikel, die in dem Innenseitenbasisteil (36) enthalten sind,
wobei die zweite Schicht (8) Folgendes einschließt:
eine Vielzahl von den Hauptrillenteilen entsprechenden Teilen (22) und eine Vielzahl von der Basis entsprechenden Teilen (24), die an Positionen angeordnet sind, die mit der Vielzahl von Hauptrillenteilen (19) bzw. der Vielzahl von Basisteilen (20) in der Dickenrichtung überlappen,
und **dadurch gekennzeichnet, dass**:
sowohl eine durchschnittliche Dichte der superabsorbierenden Polymerpartikel, die in jedem der Vielzahl von Basisteilen (20) enthalten sind, als auch eine durchschnittliche Dichte der superabsorbierenden Polymerpartikel, die in jedem der Vielzahl von den Hauptrillenteilen entsprechenden Teilen (22) enthalten sind, geringer sind als eine durchschnittliche Dichte der superabsorbierenden Polymerpartikel, die in jedem der Vielzahl von der Basis entsprechenden Teilen (24) enthalten sind.

2. Absorbierender Artikel nach Anspruch 1,
wobei eine Dicke des Außenseitenbasisteils (34) in der Dickenrichtung in einem Bereich von 20 % bis 80 % einer Dicke von jedem der Vielzahl von Basisteilen (20) in der Dickenrichtung liegt.

3. Absorbierender Artikel nach Anspruch 1 oder 2,
wobei die durchschnittliche Dichte der superabsorbierenden Polymerpartikel, die in jedem der Vielzahl von den Hauptrillenteilen entsprechenden Teilen (22) enthalten sind, geringer ist als die durchschnittliche Dichte der superabsorbierenden Polymerpartikel, die in dem Innenseitenbasisteil (36) von jedem der Vielzahl von Basisteilen (20) enthalten sind.

4. Absorbierender Artikel nach Anspruch 3,
wobei die durchschnittliche Dichte der superabsorbierenden Polymerpartikel, die in dem Außenseitenbasisteil (34) von jedem der Vielzahl von Basisteilen (20) enthalten sind, geringer ist als die durchschnittliche Dichte der superabsorbierenden Polymerpartikel, die in jedem der Vielzahl von den Hauptrillenteilen entsprechenden Teilen (22) enthalten sind.

5. Absorbierender Artikel nach einem der Ansprüche 1 bis 4,
wobei jeder der Vielzahl von Rillenteilen (18) weiter Nebenrillenteile (21) einschließt, die die erste Schicht (6) in der Dickenrichtung penetrieren und in Verbindung mit jedem der Vielzahl von Hauptrillenteilen (19) durch ein Basisende mit einem vorbestimmten Bereich in einer Richtung, die jeden der Vielzahl von Hauptrillenteilen (19) kreuzt, vorliegen.

6. Absorbierender Artikel nach Anspruch 5,
wobei die zweite Schicht (8) weiter Folgendes einschließt:
den Nebenrillenteilen entsprechende Teile (26), die an Positionen angeordnet sind, die mit den Nebenrillenteilen (21) in der Dickenrichtung überlappen, und
wobei eine durchschnittliche Dichte der superabsorbierenden Polymerpartikel, die in dem dem Nebenrillenteil entsprechenden Teil (26) enthalten sind, geringer ist als eine durchschnittliche Dichte der superabsorbierenden Polymerpartikel, die in jedem der Vielzahl von der Basis entsprechenden Teilen (24) enthalten sind.

7. Absorbierender Artikel nach einem der Ansprüche 1 bis 6,
wobei in jedem der Vielzahl von Basisteilen (20), an einem Endteil und dem anderen Endteil in der lateralen Richtung und an einem mittleren Teil zwischen dem einen Endteil und dem anderen Endteil,
eine durchschnittliche Dichte der superabsorbierenden Polymerpartikel, die in jedem des einen Endteils und des anderen Endteils enthalten sind, geringer ist als eine durchschnittliche Dichte der superabsorbierenden Polymerpartikel, die in dem mittleren Teil enthalten sind.

## Revendications

1. Article absorbant ayant une direction longitudinale, une direction latérale et une direction d'épaisseur, l'article absorbant comprenant :
un noyau absorbant (9) incluant des particules polymères superabsorbantes,
où le noyau absorbant (9) a une surface opposée à la peau et une surface faisant face à la peau et est formé d'une première couche (6) d'un côté de la surface opposée à la peau et d'une seconde couche (8) d'un côté de la surface qui fait face à la peau,
la première couche (6) inclut :
une pluralité de parties rainurées (18) incluant une pluralité de parties rainurées principales (19) qui se prolongent dans la direction longitudinale et pénètrent la première couche (6) dans la direction d'épaisseur et
une pluralité de parties de base (20) qui se prolongent dans la direction longitudinale,
chacune de la pluralité de parties rainurées principales (19) et chacune de la pluralité de parties de base (20) étant agencées d'une manière alternée,
chacune de la pluralité de parties de base (20) étant formée :
d'une partie de base latérale interne (36) qui est adjacente au côté de la surface opposée à la peau de la seconde couche (8) et
d'une partie de base latérale externe (34) qui est adjacente au côté de la surface opposée à la peau de la partie de base latérale interne (36) et
une densité moyenne des particules polymères superabsorbantes contenues dans la partie de base latérale externe (34) est inférieure à une densité moyenne des particules polymères superabsorbantes contenues dans la partie de base latérale interne (36),
où la seconde couche (8) inclut :
une pluralité de parties correspondant à une partie rainurée principale (22) et une pluralité de parties correspondant à une partie de base (24) situées à des positions qui chevauchent la pluralité de parties rainurées principales (19) et la pluralité de parties de base (20) dans la direction d'épaisseur, respectivement, et **caractérisé en ce que** :
une densité moyenne des particules polymères superabsorbantes contenues dans chacune de la pluralité de parties de base (20) et une densité moyenne des particules polymères superabsorbantes contenues dans chacune de la pluralité de parties correspondant à une partie rainurée principale (22) sont inférieures à une densité moyenne des particules polymères superabsorbantes contenues dans chacune de la pluralité de parties correspondant à une partie de base (24).

2. Article absorbant selon la revendication 1,
où une épaisseur de la partie de base latérale externe (34) dans la direction d'épaisseur est dans une plage qui représente de 20 % à 80 % d'une épaisseur de chacune de la pluralité de parties de base (20) dans la direction d'épaisseur.

3. Article absorbant selon la revendication 1 ou 2,
où la densité moyenne des particules polymères superabsorbantes contenues dans chacune de la pluralité de parties correspondant à une partie rainurée principale (22) est inférieure à la densité moyenne des particules polymères superabsorbantes contenues dans la partie de base latérale interne (36) de chacune de la pluralité de parties de base (20).

4. Article absorbant selon la revendication 3,
où la densité moyenne des particules polymères superabsorbantes contenues dans la partie de base latérale externe (34) de chacune de la pluralité de parties de base (20) est inférieure à la densité moyenne des particules polymères superabsorbantes contenues dans chacune de la pluralité de parties correspondant à une partie rainurée principale (22).

5. Article absorbant selon l'une quelconque des revendications 1 à 4,
où chacune de la pluralité de parties rainurées (18) inclut en outre des parties rainurées auxiliaires (21) qui pénètrent la première couche (6) dans la direction d'épaisseur et qui sont présentes en communication avec chacune de la pluralité de parties rainurées principales (19) par le biais d'une extrémité de base comportant un espace prédéterminé dans une direction qui traverse chacune de la pluralité de parties rainurées principales (19).

6. Article absorbant selon la revendication 5,
où la seconde couche (8) inclut en outre :
des parties correspondant à une partie rainurée auxiliaire (26) situées à des positions qui chevauchent les parties rainurées auxiliaires (21) dans la direction d'épaisseur et
une densité moyenne des particules polymères superabsorbantes contenues dans la partie correspondant à une partie rainurée auxiliaire (26) est inférieure à une densité moyenne des particules polymères superabsorbantes contenues dans chacune de la pluralité de parties correspondant à une partie de base (24).

7. Article absorbant selon l'une quelconque des revendications 1 à 6,
où, dans chacune de la pluralité de parties de base (20), au niveau d'une partie d'extrémité et de l'autre partie d'extrémité dans la direction latérale et au niveau d'une partie centrale située entre lesdites une partie d'extrémité et l'autre partie d'extrémité,
une densité moyenne des particules polymères superabsorbantes contenues dans chacune desdites une partie d'extrémité et l'autre partie d'extrémité est inférieure à une densité moyenne des particules polymères superabsorbantes contenues dans la partie centrale.
